# EUROPEAN PATENT APPLICATION

(11) **EP 3 964 133 A1**
(43) Date of publication of application: **09.03.2022**
(21) Application number: 20194504.5
(22) Date of filing: 04.09.2020
(51) Int. Cl.: A61B 6/04, A61B 6/00, G01D 5/347, G06F 3/03

(54) **METHOD AND SYSTEM FOR ENCODING A SURFACE POSITION OF AN X-RAY POSITIONING DEVICE**

(71) Applicant: AGFA NV, 2640 Mortsel (BE)
(72) Inventor: BENZINGER, Rupert, 2640 Mortsel (BE)
(74) Representative: Pieters, Dirk Paul Oscar

(57) **Abstract**

This invention is related to a method and system for a multi-axis position sensing for a floating X-ray modality table top that is based on an optical measurement of the color saturation of a combination of two distinct colors that are applied in a specific pattern on a surface of the table top. The measurement is performed by a color sensitive device that is associated with the X-ray modality table base.

## Description

### Technical Field

The present invention relates generally to the field of medical imaging modality components, wherein the position of an X-ray table top or other positioning device is determined by encoding the positional information on a surface attached to a movable surface (such as a table top) by means of a multi-color encoded scheme, such as a two (or more) color gradient.

### Background of the invention

The positioning of a patient on an imaging modality is often ensured by a patient support device such as an X-ray table, or an X-ray wall stand. An X-ray table has its patient support structure (tabletop) interposed between the patient and the image receptor during radiography or fluoroscopy. Nowadays, these support devices are motorized and allow an easy adjustment of the positioning height, and of the lateral and axial position of the patient. The positional data of the device can be read out in digital form and are required to obtain accurate positioning information on the patient, and to support various automated functions. The positional data of each movement are typically acquired by means of position encoders. A position encoder is a sensor which turns a position into an electronic signal.

In many technical applications, position encoding plays an important role in electronically driving mechanical devices. For many applications it is important to accurately determine the absolute position or the relative position of a component. Many technical approaches have been proposed and are being used nowadays to determine the physical positions of devices or components, the measuring devices being known as encoders.

Mechanical encoders or conductive encoders are based on a series of circumferential copper tracks etched onto a PCB, and are used to encode the distance information via contact brushes sensing the conductive areas. Mechanical encoders are economical but susceptible to mechanical wear.

On-axis magnetic encoders typically use a specially magnetized two pole neodymium magnet attached to the shaft of a motor, and is in principle suitable for measuring the number of revolutions of the motor shaft itself. Because the magnet can only be fixed to the end of the shaft, this principle only works with motors that have a shaft extending out of the motor body. The design of the set of gears attached to the motor shaft determines the accuracy and the type of movement that can be measured by it.

Optical encoders typically use a light shining onto a photodiode through slits in a metal, glass or transparent plastic disc or ribbon. The slits or visible marks on the ribbons or discs comprise accurate distance information that can be read by the photodiode detector. Reflective versions also exist. Optical encoders are the most commonly used type of technology. Optical encoders are however very sensitive to dust.

Distinction can be made between absolute and relative encoders. The difference between them is that an absolute encoder will produce a unique (position) reading for each position, and therefore returns absolute positional information. The reading can be produced even after the encoder has been switched off or was inactive for some time. Absolute encoders may be based on a very specific encoded pattern on for instance a transparent plastic ribbon that comprises information about the specific position when read out. An alternative technique is the use of a relative encoder of which the absolute position is calculated with reference to a so-called homing position to which the encoder has to be moved for initialisation.

In order to obtain a multi-axial positional information, or 2D positional information, it is thus common to combine multiple encoders to obtain the positional readings for each of the axes independently. The application of multiple encoders however increases the mechanical complexity of the system, and introduce additional points of possible failure. It would therefore be advantageous to reduce the number of components involved in the multi-axial position sensing configuration.

In the domain of medical imaging, the above various types of encoders are applied in the movable components of an imaging modality. The encoders are used to measure the movement of components that slide or move with respect to each other. The relative positions of all movable components of an imaging modality determine the geometric configuration of the modality. These measured movements may be a travelled distance or a rotation angle between two articulating components. In components that exhibit linear movements, it is common to apply linear optical encoders as they are less subject to wear and tear. Linear movements are for instance the up-down movement of a positioning table or overhead tube crane, the lateral movements of a table top of a positioning table, or the height of a wall stand unit, and alike.

Since most of the position encoder systems highly suffer from wear and tear, it would be an improvement if such drawbacks could be avoided. Moreover in some of the applications, a 2D encoder system would be preferred over a combination of two separate linear encoder systems as it would reduce the system complexity and the risk for mechanical breakdown.

In WO2020/031041, a multi-axis self-positioning method is described that is able to determine the absolute position of an image capturing device or read-head that is positioned over a specially encoded reference scale. The image capturing device, such as a camera, captures a limited area of the encoded reference scale. The reference scale comprises so-called de Bruijn sequences that are convolved into an array of symbols such that each neighbourhood of the encoded reference scale captured by the camera uniquely determines a unique position on said reference scale. As such, an array of symbols or half tone dots are able to encode exact absolute positional information in a small area.

### Summary of invention

The present invention provides a method and a system for multi-axis relative position sensing between a two-dimensional surface and an object such that the relative position of the object can be determined with respect to the two-dimensional surface as disclosed in Claim 1.

The present invention provides a system and method for multi-axis position sensing comprising the steps of capturing a spot color measurement of a portion of a color encoded surface by means of a color sensitive device, wherein the color encoded surface exhibits along each orthogonal axis a saturation wedge of two distinct colors, each color saturation wedge exhibiting an incremental saturation of one of said distinct colors in each direction of each of said orthogonal axes, calculating a color saturation value for each of said distinct colors from said spot color measurement, determining a multi-axis position of said spot color measurement on said color encoded surface by correlating said color saturation value for each of said distinct colors with a position along each of said axes.

In the context of this invention, multi-axis position sensing is the determination of an absolute two-dimensional position of an object with respect to a surface over which said object moves. The surface in this context may be a plane, over which an object may move in two dimensions at a constant distance from the surface, but it may as well be a curved surface such as for instance the surface of a cylindrical body over which said object may then move at a constant distance. As long as the object that moves over the plane (and of which the position is sensed) is able to maintain the constant distance from the surface that allows it to measure spot color information, distances over any surface may be sensed using the method of the invention. In this invention, the object that moves over the surface as explained above is a color sensitive device that is able to measure spot color information of the reflected light from a small area on this surface.

Light is made up of wavelengths and each wavelength has a particular colour. The colour we see is a result of which wavelengths are reflected back to our eyes. The visible spectrum showing the wavelengths of each of the component colours. The spectrum ranges from dark red at 700 nm to violet at 400 nm.

The color sensitive device of the invention is a measurement device that measures the intensity of a certain wavelength in the light reflecting from a small area on a surface that it faces. It measures the color saturation or the color intensity of the reflected light. The device is designed such that only the reflected light of a small area of the surface is captured, such that the position relating to the color measurement is accurate. The device is preferably able to measure the intensity or saturation for multiple different wavelengths, as it needs to discriminate between the distinct colors that are present in the color encoded surface. In the case that it only can detect a single wavelength or color, the application of multiple of such color sensitive devices should be considered.

The color sensitive device should thus be able to optimally detect and make a distinction between the reflected wavelength peaks from the distinct colors that are present on the color encoded surface. The colors that are picked to be applied on the color encoded surface are carefully selected to ensure that the color sensitive device is able to separate the intensity signals from each color. In order to achieve this, so-called distinct colors are chosen for application in the invention.

Distinct colors produce maximum intensity responses from reflected light at different reflection wavelengths and this without contributing to an intensity response of another of the selected distinct colors. This means that the reflection spectrum of a first distinct color should not interfere at the wavelengths producing a maximum intensity response of another distinct color. Distinct color therefore do not contribute to each others' spectrum at their maximum intensity response wavelengths. As such will the measurement of the intensity at the peak wavelength of one of the colors only contribute to the presence of said color on the color encoded surface, and not have a contributing component from another color.

In practice, the best results are achieved by selecting as a distinct color one of the three so-called main colors, i.e. cyan, magenta and yellow (from the CMY subtractive color model). These colors show clearly separated peak wavelength responses when reflecting light, and meet the above mentioned requirements.

A spot color measurement is an intensity measurement at a certain wavelength of reflected light coming from a small portion of a surface. The term spot in this context corresponds to the area that contributes to the measurement. The spot size determines the accuracy or spatial resolution of the position sensing performance. The smaller the spot, the better the higher the position sensing spatial accuracy.

The color encoded surface refers to the surface on which the color sensitive device performs its measurement to sense its position, and refers to a surface with a known shape on which a combination of distinct colors is applied. The application of the colors on the surface may be achieved by paint, ink, pigments or alike The absolute position on the surface is determined by the mix of distinct colors that is reflected at that position. The color encoding on the surface thus exhibits a unique combination of two or more selected distinct colors that uniquely determines the absolute position on the surface. The color encoding consists of at least two distinct colors that are applied at different reflection intensities in different areas.

Specific examples and preferred embodiments are set out in the dependent claims.

The invention is advantageous in that its application in devices takes up very little space, and can thus be applied in very confined spaces, such as for instance an X-ray modality table. The application of a colored surface underneath a table surface does not take up any space, while the only component taking up some space is a color sensor and a light source causing the reflected light.

Another advantage of the invention is that no moving parts are involved which may deteriorate over time, and the design only requires a single detector or sensor component to measure a position in multiple directions.

The invention is advantageous in that it provides a direct measurement of the absolute position in two dimensions.

The implementation of the invention is cost-effective compared to the solutions described in the state of the art; the implementation of the invention does not require any expensive optics.

The invention is advantageous in that when implemented in a medical imaging modality positioning table, the active components are completely integrated in the table design (and inaccessible for an operator or user), such that no loss of function can occur when for instance the edges of the table are obscured by the patient, a blanket or alike.

### Brief description of drawings

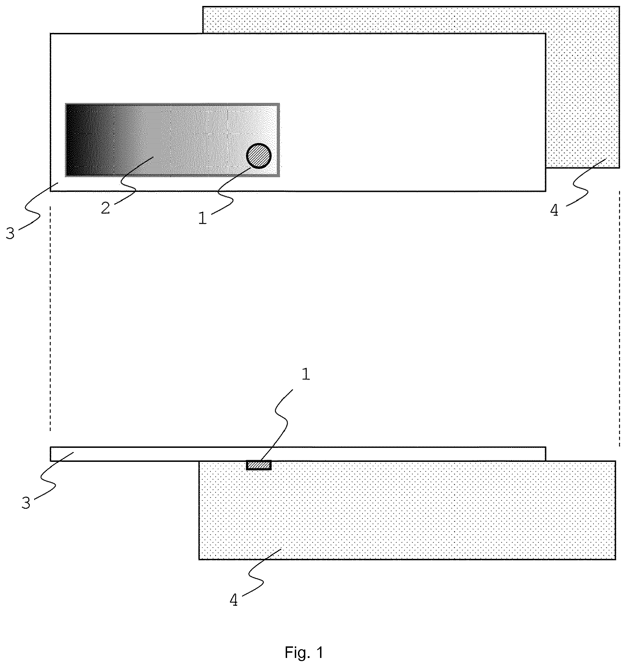

Fig. 1 gives two projections of a schematic overview of an X-ray modality table of the invention. The upper drawing is a top-down view of such a table, whereas the bottom drawing is the lateral view. In both drawings the table top surface [3] is visible, and the table base [4] as well. The rectangle [2] represents the color encoded surface that is applied at the underside of the table top [3], and that is in principle not visible in this perspective. The color encoded surface [2] exhibits color encoding (here a linear saturation wedge) that can be measured by the color sensing device [1]. The color sensitive device [1] is integrated in the base of the table [4], as can be seen in the bottom drawing.

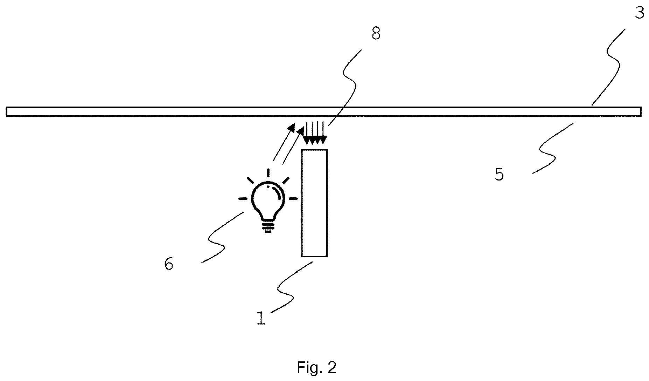

Fig. 2 is a schematic overview of a detail of the X-ray modality table of the invention, and illustrates that the color sensitive device [1] captures the reflected light [8] from the color encoded surface at the underside of the modality table [5]. A light source [6] provides sufficient light such that the reflected light can be measured.

### Description of embodiments

In the following detailed description, reference is made in sufficient detail to the above referenced drawings, allowing those skilled in the art to practice the embodiments explained below.

The multi-axis position sensing thus relates to the determination of the absolute displacement of one object with respect to a surface. Bringing this concept into the context of an X-ray modality table top, the position sensing of a movable table top with respect to the (fixed) position of the X-ray table base may be considered as a good example. The invention is however not limited to the application in an X-ray modality table, and may also be applied in other modality components, such as in a detector wall stand or alike, and even in other engineering contexts or industrial applications. The invention is generally suitable for applications where an object floats at a close distance over a surface.

In this invention, there are two essential components that interact with each other to determine the position information that uniquely determines the location of one object with respect to a surface (or the object associated with this surface). These two components are a color sensing device and a color encoded surface. The method allows to measure the respective position between these two components based on color intensity or color saturation values measured by the color sensitive device of a small portion of the color encoded surface that is located in front of the color sensitive device.

The first essential component is a color sensitive device which is positioned such that it efficiently can capture light that reflects from a portion of the color encoded surface. In practice, the color sensitive device will be mounted such that it hovers over the color encoded surface at a fixed distance at any given position. If a fixed distance cannot be observed, compensations will have to be taken into account to adjust the measurements.

It speaks for itself that the total area of the surface on which the position sensing can be performed is limited to the actual size of the color encoded surface itself. Depending on the spotsize of the color sensing measurement, some distance may have to be observed from the borders of the encoded surface in order to ensure reliable measurements; the position sensing only works correctly when the color sensitive device exclusively measures the reflected light signal from the color encoded surface. In case that a measurement would be influenced by the color of a border around the color encoded surface, incorrect position sensing would result.

The color of reflected light of a portion of the color encoded surface is preferably measured using a spectrophotometer (also called spectro-reflectometer or reflectometer). A spectrophotometer has the advantage that it can measure the intensity of the incoming reflected light at different wavelengths. Other color sensitive devices may be limited to measure the intensity of the incoming light only at a single wavelength, or may have a non-linear response at different wavelengths in a certain wavelength range.

A spectrophotometer takes measurements of the reflected light in the visible region (and a little beyond) of a given color sample and at a certain selected wavelength. The spectrophotometer will take one measurement at a time of the amount of reflected light at a certain selected wavelength. As such, the spectrophotometer can measure the intensity of reflected light at different wavelengths (and thus of different colors). It is also possible to scan the entire range of visible light at a number of increments such that the sample's spectral reflectance curve is obtained. If, for instance, the custom of taking readings at 10 nanometer wavelength increments is followed, the visible light range of 400-700 nm will yield 31 readings, making up the sample's spectral reflectance curve.

The color sensitive device preferably outputs its measurement result as a digital value. It is on today's market not very difficult to find an integrated low cost color sensor that outputs its measurements as an 8- or 16-bit value. The color sensor is in this case integrated with a digital data interface that can be read out by in a convenient way. The true bit-depth of the output by the sensor (not of the output format) will determine the accuracy of the measured values. In case that the accuracy of the sensor is 10 bits, there will be upto 1024 different color intensities that may be detected. This would lead to a spatial accuracy of 1mm in case that the full color range would be distributed over the distance of 1 meter (1 meter divided by about 1000 values corresponds to 1mm per measurement value increment).

In order to be able to obtain a reliable measurement signal from the color sensing device, a sufficient amount of reflected light has to be generated by a light source in order to guarantee a sufficient amplitude of the signal. In principle, this light could come from ambient light that is present in the operating environment, but would preferably be generated by a dedicated artificial light source. In order to ensure a sufficiently stable and reproducible measurement signal, it is advisable to provide an artificial light source of which the intensity and color is controlled and sufficiently stable. The intensity of the light source will directly determine the amount of reflected light into the color sensitive device, whereas the color of the light source will determine which wavelengths will be reflected by the color encoded surface. It therefore is preferred to foresee a white light source that produces a sufficient amount of reflected light at all wavelengths of the color spectrum. Alternatively, the light source has to emit a color spectrum that allows clear separation of the two distinct colors after refraction to the color sensing device. For instance, the use of an RGB LED as a light source provides the advantage of producing a selectable narrow spectrum that can be adapted to a particular color sensor that, for instance, may filter out all but the narrow color band produced by the LED.

Also measured sensitivity in blue is much lower then e.g. in red so sensitivity difference can be compensated by light source.

Given that the color sensitive device will be mounted such that it hovers over the color encoded surface at a fixed distance at any given position, the shape of the surface itself is preferably a plane or a cylinder over which a constant distance can be easily maintained.

The color encoded surface is thus a -preferably flat- surface on which the color encoding is applied. This may be achieved by directly applying paint, ink or pigments, by means of ink jetting, spray painting or any other suitable dye application method that produces a stable colored paint coating. A base color layer may be applied first directly to the surface itself as a primer. This primer layer is preferable black or white, as this will make the application of the colored patterns easier and provide a better result.

Alternatively, the color encoding may be applied onto an organic or synthetic base film or base layer that then in its own turn will be applied, fixed or glued onto the surface. The base film or layer will preferable have an intrinsic solid black or white color such that the application of the colored patterns are easier and provide a better result. The material for this film may be any organic or synthetic polymer film (such as for instance PET, PVC or alike) that may be self-adhesive. Alternatively a paper sheet may be used as a base layer.

A next aspect of the color encoded surface is the type of pattern that will be applied by combining the at least two distinct colors. The most simple embodiment of this color encoding would be to have two linear color intensity wedges of the two distinct colors superimposed onto each other, but where the wedge directions for the two distinct colors are orthogonal to each other. This means that a first linear color saturation (or intensity) wedge would be oriented for instance in the longitudinal direction of the (rectangular) surface, exhibiting thus a linear color saturation increase starting from black or white at one edge, in order to increase to a maximum color saturation of one of the distinct colors at the opposing edge. The wedge would thus range from full black/white to -for instance-full cyan/magenta/yellow. In the orthogonal direction to the previous wedge, a second linear wedge would then be applied based on a different distinct color than the first color wedge.

In this case, both color wedges are superimposed onto each other such that an exact and unique combination of color intensities or color saturations at a certain position uniquely determines a position on the color encoded surface. The two orthogonally oriented color wedges are superimposed onto each other, meaning that the two distinct colors will be superimposed on each other on the most part of the surface. Both distinct colors will thus contribute to a mix of both colors, which will result in all variations of colors in the color space that are located between their most extreme values, namely the two distinct colors themselves.

While the preferred embodiment of an applied pattern for a color encoded surface indeed would be a set of superimposed color wedges, different patterns would be conceivable as well. It would for instance be possible to randomly distribute the different colors (the different color mixes of the two distinct colors) that are discernible by the color sensitive device across the surface, and store a map of said different colors and their respective positions in a memory, such that still by measuring the color saturation of both colors at a certain location it would be possible to derive the position of the color measurement.

In order to further expand the usable range of the surface, more color combinations are required. Adding a third distinct color will provide additional color saturation values. Adding a third distinct color to the color mix actually ads an additional color channel that multiplies the number of possible color combinations of the original two distinct colors with the amount of measurable color saturation values for the third distinct color. In other words, the number of possible color combinations increases from *n*² to *n*³*,* in the assumption that *n* is the number of discernable color saturation values that can be detected by the color sensitive device.

In a last step, the measured color saturation values for the distinct colors, that are obtained from the measurements from the color sensitive device, have to be brought into relation with the exact location on the encoded surface. Depending on the choice of the pattern that is applied as the color encoding on the surface, can the exact location of the color reading from the color sensitive device be determined by a calculation or by a lookup action in a table.

In the case that color saturation values have for instance a linear relation with their location on the surface, can the location be calculated based on the measured color saturation values.

In an alternative embodiment, the location on the surface is determined by a color saturation ratio between two color pairs of the distinct colors on the color encoded surface. So, in one direction, a gradient between a first color pair is applied, and in the other direction a gradient between a second color pair is applied. Each position on the color encoded surface thus will have a unique set of two color pair ratios, allowing to uniquely define a ratio combination for each position. In this embodiment, a total of at least three different distinct colors will need to be applied in order to obtain two different color saturation ratios in both spatial directions. Three different distinct colors allow to have two color pairs to construct two different color ratios; one of the colors is then used in bot color pairs. This alternative embodiment has the advantage that the measurements are less dependent on the light source quality and intensity, and provide more stable location measurement results. Moreover, the application of these color ratios intrinsically compensates for the deterioration of the light source quality and the color sensor quality over time.

In the case that a more complex pattern is used that distributes the unique color combinations across the surface, a lookup table associating the color saturation values with their respective position on the color encoded surface may be applied. This lookup table can be created by scanning all positions on the surface and recording the color saturation values at each position, and storing these values together with the respective position in a computer memory.

As stated earlier, the invention can be applied in devices where relative positions have to be determined of two parts that move with respect to each other. One of the objects should comprise a surface about which the relative movement should be tracked. This surface should allow the application of the color encoding, which means that it should be possible to apply the encoded coloring on this surface at the time of production.

An example of such a device is a patient positioning table that can be used in an X-ray environment or in another medical context. Such a positioning table comprises at least a positioning table top that may have a flat surface, but in most cases has a flat underside. This underside can then be used as the color encoded surface of the invention.

The table top of a positioning table can freely move in two directions relative to its table base in a longitudinal (X-direction) and a lateral (Y-direction) direction. The up- and down-movement of the table is managed by the table base itself. The X- and Y-direction movement will be tracked by the method of the invention.

In order to track the movement of the table top relative to the table base, a color sensitive device is mounted in the table base, and this with its sensor optics oriented towards the underside of the table top. As such, can the color sensitive device measure the portion of the color encoded surface that is right in front of it. When the table top position is altered in X- or Y-direction, the color sensitive device will be positioned in front of a different portion of the surface at the underside of the table top.

The invention may be applied in other applications such as for instance in the position tracking of a detector wall stand, but also in other non-medical applications. The position of a print head or CNC-device may be determined by this same technique on condition that the relative position with respect to a non-moving surface can be associated to a color encoding on this surface. In this case, a color sensitive device should be physically associated with the print head or the CNC-driven tool.

## Claims

1. Method for multi-axis position sensing comprising the steps of:
- capturing a spot color measurement of a portion of a color encoded surface by means of a color sensitive device, wherein the color encoded surface exhibits a unique combination of color saturation values of at least two distinct colors at each portion of said color encoded surface, and of which each combination of color saturation values is stored with corresponding location data of said portion on said color encoded surface in a lookup table in a memory ,
- calculating a color saturation value for each of said distinct colors from said spot color measurement,
- determining a multi-axis position for said spot color measurement on said color encoded surface by retrieving the position on said color encoded surface from a lookup table in said memory that corresponds to said color saturation values.

2. Method for multi-axis position sensing comprising the steps of:
- capturing a spot color measurement of a portion of a color encoded surface by means of a color sensitive device, wherein the color encoded surface exhibits along each orthogonal axis a saturation wedge of two distinct colors, each color saturation wedge exhibiting an incremental saturation of one of said distinct colors in each direction of each of said orthogonal axes,
- calculating a color saturation value for each of said distinct colors from said spot color measurement,
- determining a multi-axis position of said spot color measurement on said color encoded surface by correlating said color saturation value for each of said distinct colors with a position along each of said axes.

3. Method for multi-axis position sensing of a floating X-ray modality table top according to Claim 1 or Claim 2, wherein said two-dimensional color scale is applied on a surface of said table top, and where said color sensitive device is fitted to a base of said X-ray modality table.

4. Method for multi-axis position sensing of a floating X-ray modality table top according to Claim 1 or Claim 2, wherein said multi-axis position on said color encoded surface is determined by calculating two ratios between two color saturation value pairs that are obtained from said spot color measurements on said color encoded surface, said ratios being correlated to said multi-axis position.

5. Method according to Claim 3, wherein said two-dimensional color scale is applied on a surface of said table top opposing the surface on which a patient is positioned.

6. Method according to Claim 1 or Claim 2 wherein said color sensitive device is a colorimeter.

7. Method according to Claim 2 wherein said two-dimensional color encoded surface comprises a series of collated saturation wedges to extend the measurement span.

8. Method according to Claim 1 or Claim 2 wherein the color encoded surface comprises additionally patterns or symbols to reflect distinction between collated zones.

9. Method according to Claim 1 or Claim 2 wherein the measured portion of said color encoded surface is illuminated by a light source in order to optimize the color signal readings.

10. System for multi-axis position sensing for a floating X-ray modality table top, comprising:
- a color encoded surface that is applied on one side of a surface of said table top, and that exhibits a unique combination of color saturation values of at least two distinct colors at each portion of said color encoded surface, and of which each combination of color saturation values is stored with corresponding location data of said portion on said color encoded surface in a lookup table in a memory,
- a color sensitive device that is positioned in front of said color encoded surface configured to perform a color spot measurement on a portion of said color encoded surface,
- a memory,
- a processing unit, that calculates a color saturation value for each of said distinct colors from said spot color measurement, and that determines a multi-axis position for said spot color measurement on said color encoded surface by retrieving the position on said color encoded surface from a lookup table in said memory that corresponds to said color saturation values.

11. System for multi-axis position sensing for a floating X-ray modality table top, comprising:
- a color encoded surface that is applied on one side of a surface of said table top, and that exhibits along each orthogonal axis a saturation wedge of two distinct colors, each color saturation wedge exhibiting an incremental saturation of one of said distinct colors in each direction of each of said orthogonal axes,
- a color sensitive device that is positioned in front of said color encoded surface configured to perform a color spot measurement on a portion of said color encoded surface,
- a processing unit, that determines a multi-axis position from said spot color measurement on said color encoded surface by correlating said color saturation value for each of said distinct colors with a position along each of said axes.
